# EUROPEAN PATENT SPECIFICATION

(11) **EP 2 427 223 B1**
(45) Date of publication and mention of the grant of the patent: **18.03.2015**
(21) Application number: 10719834.3
(22) Date of filing: 03.05.2010
(51) Int. Cl.: A61L 15/20, A61L 15/46

(54) **FEMININE HYGIENE ARTICLE HAVING CALCIUM SUGAR ACID SALT**
DAMENHYGIENEARTIKEL MIT CALCIUM-ZUCKERSÄURE-SALZ
ARTICLE D'HYGIÈNE FÉMININE COMPORTANT UN SEL D'ACIDE DE SUCRE DE CALCIUM

(30) Priority: 05.05.2009 US 435608
(43) Date of publication of application: 14.03.2012
(73) Proprietor: The Procter & Gamble Company, Cincinnati, OH 45202 (US)
(72) Inventor: NEMETH, Kimberly, Ann, Mason Ohio 45040 (US); OSBORN, Thomas, Ward, III, Cincinnati Ohio 45220 (US); WANG, Fancheng, Blue Ash Ohio 45242 (US)
(74) Representative: Kremer, Véronique Marie Joséphine
(86) International application number: PCT/US2010/033325
(87) International publication number: WO 2010/129443

(56) References cited:
- WO-A1-86/05388
- WO-A1-2009/003073
- US-A- 5 389 374
- US-A1- 2005 033 255

## Description

### FIELD OF THE INVENTION

The present invention relates to feminine articles having one or more calcium sugar acid salts, wherein the calcium sugar acid salts reduce the production of Toxic Shock Syndrome Toxin-1.

### BACKGROUND OF THE INVENTION

*Staphylococcus aureus (S. aureus*) is a bacterium that commonly colonizes human skin and mucous membranes. It causes disease in humans through the production of toxic proteins. One such disease is Toxic Shock Syndrome (TSS), caused by Toxic Shock Syndrome Toxin-1 (TSST-1) and other similar toxins. When absorbed into the blood stream, TSST-1 may lead to TSS in non-immune humans. It has been thought that TSS is associated with growth of S. *aureus* in the presence of tampons, such as those used in nasal packing or as catamenial devices. *S. aureus* is found in the vagina of approximately 10% of healthy women of menstrual age. Approximately 1% of the S. *aureus* isolates from the vagina are found to produce TSST-1.

Symptoms of TSS generally include fever (>102°F), diarrhea, vomiting, a sunburn-like rash, hypotension, and multiple organ failure. As S. *aureus* grows and multiplies, it can produce TSST-1. In some cases, only after entering the bloodstream does TSST-1 toxin act systemically and produce the symptoms attributed to TSS.

When S. *aureus* is present in an area of the human body that harbors a normal microbial population such as the vagina, it may be difficult to eradicate the S. *aureus* bacteria without harming members of the normal microbial flora required for a healthy vagina. Typically, antibiotics that kill *S. aureus* are not a viable option for use in feminine articles. This is because of their effect on the normal vaginal microbial flora and their tendency to stimulate toxin production in the *S. aureus* that are not killed. An alternative to eradication is technology designed to prevent or substantially reduce the bacteria's ability to produce toxins.

There have been attempts in the prior art to reduce or eliminate pathogenic microorganisms and menstrually occurring TSS by incorporating into a feminine article one or more biostatic, biocidal, or detoxifying compounds. For example, L-ascorbic acid has been applied to a tampon to detoxify toxin found in the vagina. Other examples have incorporated into tampons monoesters and diesters of polyhydric aliphatic alcohols, such as glycerol monolaurate, as potential biocidal compounds. Still further examples have introduced to tampons non-ionic surfactants, such as alkyl ethers, alkyl amines, and alkyl amides as detoxifying compounds.
US 2005/0033255 A1 discloses absorbent structures that comprise water-swellable polymers that are coated with a coating agent comprising a phase-separating elastomeric material.
WO 2009/003073 A1 discloses an article comprising one or more calcium salts to reduce the production of TSST-1 by at least 50% when measured by the Shake Flask Method.

Despite the above mentioned attempts, there continues to be a need for compounds that will effectively inhibit the production of TSST-1, without impacting the normal vaginal flora. Further, it is desirable that the TSST-1 reducing compounds are non-harmful to S. *aureus*.

### SUMMARY OF THE INVENTION

A feminine article is provided that comprises a calcium sugar acid salt. The calcium sugar acid salt reduces TSST-1 production of S. *aureus* by at least about 70%, as measured by ELISA, as compared to TSST-1 production by S. *aureus* to which no calcium sugar acid salt has been added, wherein the calcium sugar acid salt has a solubility that provides a Ca concentration of at least about 50 millimoles/L of water at 25°C, wherein the feminine article is an absorbent article comprising one or more absorbent materials that absorb or contain a bodily fluid, and wherein the calcium sugar acid salt is added to fiber forming the absorbent material.

The feminine article may comprise a D-form calcium sugar acid salt. The D-form calcium sugar acid salt reduces S. *aureus* TSST-1 production as measured by ELISA, as compared to TSST-1 production by S. *aureus* to which no D-form calcium sugar acid salt has been added.

### DETAILED DESCRIPTION OF THE INVENTION

The present invention is directed to feminine articles comprising one or more calcium sugar acid salts. The vaginal environment is host to a variety of microorganisms, some of which are potential pathogens. One potential pathogen is the bacteria *Staphylococcus aureus (S. aureus*). *S. aureus* can produce Toxic Shock Syndrome Toxin-1 (TSST-1), which has been associated with the onset of Toxic Shock Syndrome. The present invention, of hygiene articles comprising calcium sugar acid salts, reduces the amount of TSST-1 produced by S. *aureus* without significantly affecting the growth of S. *aureus*.

As used herein, the term "feminine article" includes absorbent articles and non-absorbent articles intended to be worn in or near the vagina. Absorbent feminine articles can be worn by women for menstrual or light incontinence control, for example tampons, sanitary napkins, intralabial devices, incontinence articles, and liners. Non-absorbent feminine articles can include pessaries for the treatment of vaginal prolapse or incontinence, cervical caps, contraceptive sponges, menstrual cups, or contraceptive diaphragms.

As used herein, the term "absorbent article" refers to devices comprising one or more absorbent materials that absorb or contain a bodily fluid, such as urine, blood, or menses. A typical absorbent article can be placed within, against or in proximity to the body of the wearer to absorb and contain various bodily fluids.

The term "absorbent material" as used herein can be constructed from a wide variety of materials commonly used in absorbent articles. Such materials include, but are not limited to synthetic fibers, natural fibers, or combinations thereof, which may be used to produce a fiber matrix. The natural fibers may include, but are not limited to, cotton, wood pulp, flax, hemp, and rayon, such as GALAXY Rayon (a tri-lobed rayon structure) available as 6140 Rayon; or SARILLE L rayon (a round fiber rayon), both available from Kelheim Fibers GmbH of Kelheim, Germany, cotton, wood pulp, flax, and hemp. The synthetic fibers can include, but are not limited to, fibers such as polyester, polyolefin, nylon, polypropylene, polyethylene, polyacrylic, vinyl polyacetate, polylactic acid, polyhydroxyalkonate, polyglycolic acid, polyacrylate, styrene block copolymers, cellulose acetate, or bicomponent fibers, such as bicomponent polyethylene and polypropylene fibers. Additional absorbent material include materials such as, peat moss, capillary channel fibers (such as those disclosed in U.S. Patent No. 5,356,405), high capacity fibers (such as those disclosed in U.S. Patent No. 4,044,766), superabsorbent polymers or absorbent gelling materials (such as those disclosed in U.S. Patent No. 5,830,543), may be incorporated into the tampon. Further, absorbent materials may comprise absorbent foams. Absorbent foams may include materials such as polyurethane, melamine-formaldehyde, polyvinyl alcohol-formaldehyde, or cellulose. Another example of absorbent foams that can be used in the present invention are foams that include polymeric foam materials that result from the polymerization of certain water-in-oil emulsions having therein a relatively high ratio of water phase to oil phase. Emulsions of this type, which have these relatively high water to oil phase ratios are known in the art as high internal phase emulsions ("HIPEs" or "HIPE" emulsions). The polymeric foam materials that result from the polymerization of such emulsions are referred to as "HIPE foams." Examples of HIPE foams are found in U.S. Pat. No's 5,260,345; 5,387,207; 5,817,704; 5,550,167; 5,827,909; 6,365,642; 6,369,121; 6,525,106; 6,362,244.

As used herein, the term "tampon" refers to any type of absorbent article that is inserted into the vaginal canal for the absorption of fluid therefrom. Typically, tampons are constructed from an absorbent material, which may be in the form of a pledget that has been compressed into a vaginally insertable shape.

As used herein, the term "pledget" refers to a construction of absorbent material prior to the compression of such construction into a tampon. A pledget can have a variety of shapes, including but not limited to, oval, round, chevron, square, rectangular, trapezoidal, and the like. To produce a tampon, a pledget may be compressed into a generally cylindrical configuration in the radial direction, axially along the longitudinal axis or in both the radial and axial directions.

A pledget may include one or more overwraps. The overwrap can be any suitable material, such as, for example, rayon, cotton, bicomponent fibers, polyethylene, polypropylene, polylactic acid, polyhydroxyalkonate, polyglycolic acid, styrene block copolymers, other suitable natural or synthetic fibers known in the art, and mixtures thereof. In certain embodiments, a pledget can comprise an overwrap material that substantially encloses the pledget. In certain embodiments, a pledget can include an overwrap material that extends beyond the withdrawal end and forms a finger cover or absorbent skirt.

A pledget can additionally include a withdrawal member. The withdrawal member can be any suitable configuration, such as one or more cords, strings, finger covers, ribbons, an extension of a material of the device, or combinations thereof. The withdrawal member can be made of any suitable material, such as cotton or rayon. The withdrawal member can optionally be provided with a secondary absorbent member, such as a mass of secondary absorbent material attached to the withdrawal member proximate the withdrawal end of the pledget. Examples of secondary absorbent members that may be used are described in U.S. Patent No. 6,258,075.

As used herein the term substantially "non-lethal" with regard to *S. aureus* means the cell density of *S. aureus* in the test fluid containing a calcium sugar acid salt and *S. aureus* grown using the Shake Flask Method is not reduced by more than a factor of about 10² CFU/ml (2 log) of test fluid relative to the *S. aureus* grown in the absence of the calcium sugar acid salt using the Shake Flask Method.

As used herein the term substantially "lethal" with regard to *S. aureus* means the cell density of test fluid containing a calcium sugar acid salt and *S. aureus* grown using the Shake Flask Method is reduced by a factor of at least about 10³ CFU/mL (3 log) relative to test fluid containing *S. aureus* grown using the Shake Flask Method, but not containing a calcium sugar acid salt.

As used herein, the term "stable" means the calcium sugar acid salt can have TSST-1 reducing capability when exposed to environmental conditions, such as water vapor (humidity) or temperatures above or below about room temperature (temperatures above or below about 25°C) during manufacture or storage.

As used herein the term "fugitive" means the calcium sugar acid salt is capable of moving through the fiber matrix of the absorbent material of an absorbent article.

As used herein, the term "bound" means less than about 10% of the calcium added to an absorbent article is removed by soaking the absorbent article over an 8 hour period at 37°C in three times the syngyna capacity of sterile physiologic saline solution. The syngyna capacity is determined by the syngyna test (U.S. FDA 21 CFR 801.430, Revised as of April 1, 2006). The percent of "bound" calcium is calculated as (calcium present in the article - calcium in solution) divided by calcium present in the article.

As used herein, the term "partially bound" means less than about 50% of the calcium added to the absorbent article is removed by soaking the article over an 8 hour period at 37°C in three times the syngyna capacity of sterile physiologic saline solution.

As used herein, the term "substantially bound" means less than about 25% of the calcium added to the absorbent article is removed by soaking the article over an 8 hour period at 37°C in three times the syngyna capacity of sterile physiologic saline solution.

In certain embodiments, calcium sugar acid salts are calcium salts of sugar acids derived from cyclic and non-cyclic sugars, such as aldoses and ketoses, having from about 3 carbon atoms to about 14 carbon atoms (C₃-C₁₄). In certain embodiments, calcium sugar acid salts are calcium salts of sugar acids derived from sugars involved in glycolysis. Examples of calcium sugar acid salts include calcium glucarates, calcium gluconates; calcium diketogluconates, such as calcium 2, 3-diketogluconate; calcium heptagluconates, such as calcium α-D-heptagluconates; calcium glycerates, calcium fructonates, calcium altronates, calcium mannonates, calcium gulonates, calcium galactonates, calcium ribonates, calcium arabinonates, calcium xylonates, calcium erythronates, calcium sorbonates, calcium ribulonates, and calcium xylulonates.

In certain embodiments, calcium sugar acid salts may have the following chemical structure: 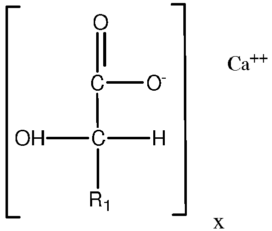 Wherein:
R₁= (CHOH)ₙCH₂OH; (CHOH)ₙCHO; (CH₂OH)ₙCOO; (CHOH)ₙCH₂(O)R₂
R₂= H; (CHOH)ₘCH₂OH
n=0-4
m=0-3
m+n= 0-3
x= 1 or 2

Examples of calcium sugar acid salts include:

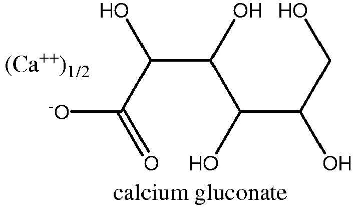

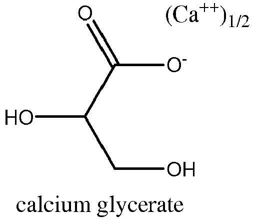

In certain embodiments the D-form of a calcium sugar acid salt is used in the hygiene article to reduce the production of TSST-1 by *S. aureus*. An optical isomer, such as the D-form of a calcium sugar acid salt, can be named by the spatial configuration of its atoms. The D/L system does this by relating the molecule in question to glyceraldehyde. Glyceraldehyde is chiral itself, and its two isomers are labeled D and L. Therefore calcium sugar acid salts are named D or L based upon the spatial configuration of their atoms as compared to glyceraldehyde.

In certain embodiments, a feminine article can include a unit dose of one or more calcium sugar acid salts. In certain embodiments, a unit dose is an amount of one or more calcium sugar acid salts that are added to a feminine article to provide a desired reduction in TSST-1 (as compared to TSST-1 produced by *S. aureus* to which no calcium sugar acid salt was added). In certain embodiments, the unit dose of calcium sugar acid salt is an amount suitable to provide the hygiene article with greater than about 0.002 millimoles of calcium, greater than about 0.004 millimoles calcium, greater than about 0.006 millimoles calcium, greater than about 0.008 millimoles calcium, greater than about 0.01 millimoles calcium, greater than about 0.02 millimoles calcium, greater than about 0.04 millimoles calcium, greater than about 0.06 millimoles calcium, greater than about 0.08 millimoles calcium, greater than about 0.1 millimoles calcium, greater than about 0.5 millimoles calcium, greater than about 1 millimoles calcium, greater than about 2 millimoles calcium, greater than about 3 millimoles calcium, greater than about 4 millimoles calcium, greater than about 5 millimoles calcium, greater than about 6 millimoles calcium, greater than about 7 millimoles calcium, greater than about 8 millimoles calcium, greater than about 8 millimoles calcium, greater than about 9 millimoles calcium, greater than about 10 millimoles calcium or more. In certain embodiments, less than substantially all of the calcium sugar acid salt that is added to the article is available to reduce the production of TSST-1, for example when some of the calcium sugar acid salt is retained within the feminine article during use. The production of TSST-1 by *S. aureus* to which one or more calcium sugar acid salts have been added, can be reduced by any suitable amount, such as by at least about 70%, 80%, 90%, 95%, or more, as compared to TSST-1 production by S. *aureus* to which no calcium sugar acid salt has been added. In certain embodiments, the amount of TSST-1 produced by S. *aureus* can be measured using the *Enzyme*-*Linked ImmunoSorbent Assay* (ELISA) technique.

In addition, the one or more calcium sugar acid salts can be substantially non-lethal to S. *aureus*, as demonstrated when S. *aureus* is grown using the shake flask method, and the colony forming units (cfu) determined.

The calcium sugar acid salt has a solubility that provides a Ca concentration of at least about 50 millimoles/L of water at 25°C, at least about 60 millimoles/L of water at 25°C, at least about 70 millimoles/L of water at 25°C, at least about 80 millimoles/L of water at 25°C, or more.

In certain embodiments, the TSST-1 reducing properties of calcium sugar acid salt will not be substantially diminished because of the tampon making process, tampon packaging, or tampon storage. For example, the calcium sugar acid salt can retain its ability to inhibit the production of TSST-1 following a tampon making process or when stored in conditions, such as high or low temperatures related to the commercial manufacture and sale. In certain embodiments, the calcium sugar acid salt can retain its ability to inhibit the production of TSST-1 for any suitable time at any suitable temperature, such as at about 100°C for about 3 hours, from about -30°C to about 65°C for about 24 hours, from about 0°C to about 50°C, or any other suitable temperature.

In certain embodiments, the calcium sugar acid salt can resist acquisition of moisture in a humid environment. Absorbent articles, such as tampons can be exposed to conditions where the relative humidity can exceed 80%, for example during commercial storage or in the bathroom during a shower. Some salts, such as calcium chloride, magnesium chloride, and zinc chloride, are deliquescent substances that can have a strong affinity for moisture and can absorb a relatively large amount of fluid from the atmosphere. The use of a deliquescent substance in a tampon can result in moisture being drawn into the tampon, such that the tampon can expand, causing difficulties with an applicator during insertion into the body or expulsion therefrom.

A unit dose of calcium sugar acid salt can be added to a feminine article by any suitable process or at any step in the feminine article manufacturing or packaging process. The calcium sugar acid salt can be added to one or more portions of a feminine article. One such example can be a tampon having the calcium sugar acid salt added into or on the primary absorbent member, the overwrap, the secondary absorbent member, the withdrawal member, or combinations thereof. In certain embodiments, the calcium sugar acid salts can also be added on, within or throughout one or more portions of the tampon.

In certain embodiments, a calcium sugar acid salt can be added to a feminine article or a portion thereof, such as fibers using one or more pharmaceutically acceptable and compatible carrier materials. Some suitable examples of carrier materials include aqueous solutions, gels, suspensions, foams, lotions, balms, salves, ointments, boluses, suppositories, or combinations thereof. In certain embodiments, it is also possible to add the calcium sugar acid salt as a powder. The calcium sugar acid salt is added to the fiber forming the absorbent material. The calcium sugar acid salt can be added directly into the fiber during manufacturing of the fiber. In certain embodiments, such as, for example when using polyethylene fibers, polypropylene fibers, polyethylene terephthalate fibers, conjugate fibers, bicomponent fibers, rayon fibers, or any other suitable synthetic fibers, the calcium sugar acid salt can be added to the melt prior to the formation of the fibers. As the resulting fibers cool, the calcium sugar acid salt can migrate to the surface of the fiber. In certain embodiments, a unit dose of calcium sugar acid salt can be added such that the amount of calcium that migrates to the surface of the fiber is sufficient to reduce TSST-1 production. The concentration of the calcium sugar acid salt added to the polymer melt can be any suitable concentration, for example between about 10% and about 30% or between about 15% and about 25% of the fiber weight.

In certain embodiments, a calcium sugar acid salt can be added to a fiber during a feminine article forming process, such as the tampon making process prior to forming the pledget, for example, in the fiber washing and drying steps or when a fiber finishing agent is added to facilitate fiber processing. In addition, a calcium sugar acid salt can be added to a fiber after the pledget is made. For example, a calcium sugar acid salt can be added to one or more layers of a pledget prior to compression by exposing one or more portions of the pledget to an aqueous solution or suspension containing a calcium sugar acid salt. Examples of methods for exposing a tampon pledget to an aqueous solution include, for example spraying the aqueous solution on the pledget, dipping the pledget in the aqueous solution or washing the pledget with the aqueous solution. Alternatively, or in addition, a calcium sugar acid salt can be incorporated in the tampon after compression, such as, for example by exposing a substantially completed tampon to an aqueous solution containing the calcium and then drying the tampon.

While the distribution of the calcium sugar acid salt on or within a feminine article of the present invention, such as a tampon, can vary as needed, in certain embodiments, the calcium sugar acid salt present in the one or more portions of the hygiene article can be distributed such that suitable effectiveness for reducing or prohibiting the production of TSST-1 on or within the feminine article can be attained. The calcium sugar acid salt included in the one or more portions of a feminine article of the present invention can be fugitive, loosely adhered, bound, partially bound, substantially bound, or any combination thereof.

A feminine article of the present invention can optionally include other beneficial components commonly found in pharmaceutical compositions, such as, for example vitamins, herbs, aloe, moisturizers, botanicals, supplementary antimicrobials, anti-parasitic agents, antipruritics, astringents, local anesthetics, or anti-inflammatory agents. In certain embodiments, the calcium can work in conjunction with one or more of the optionally included components in a complementary or synergistic way.

The present invention is further illustrated by the following examples, which should not be construed as limiting in any way.

### EXAMPLES

### EXAMPLE 1

This example demonstrates the reduction of the amount of TSST-1 as measured by the *Enzyme-Linked ImmunoSorbent Assay* (ELISA), produced by *S. aureus* grown using the shake flask assay, upon the addition of low concentrations, less than about 5 mM Ca, of calcium sugar acid salts of the present invention.

### Materials and Methods

### Shake Flask Method

The Shake Flask Method was performed in triplicate with appropriate controls. Twenty-five mL of Brain Heart Infusion broth (BHI) (Difco-BD Biosciences, Bedford, MA) was dispensed into 250 mL flasks and the flasks were covered. The medium was autoclaved at 121°-124°C for 15 minutes and allowed to cool to room temperature.

A calcium sugar acid salt solution was prepared by dissolving an appropriate amount of calcium sugar acid salt into 2.5 mM Phosphate Buffered Saline (PBS)/150 mM sodium chloride, pH 7.0. The calcium sugar acid salt solution was added to the BHI medium supplemented with 1% yeast extract at a volume ratio of 25mL:25mL. Each dilution was tested in triplicate. Each dilution was added to a 250mL Erlenmeyer flask. The flasks were inoculated with approximately 10⁶ CFU/mL of an 18-24 hour culture of S. *aureus* MN 8 then incubated at 37°C in ambient air supplemented with 5% CO₂ while shaking for 18-20 hours. Culture fluid was streaked onto a Blood Agar Plate to ensure purity. A corresponding control of PBS and BHI was also tested. The flasks were removed from the shaker and a 3-4mL sample of fluid was aseptically removed. A standard plate count analysis and ELISA were performed using standard techniques. Results for the test solutions were compared to the appropriate control.

### Enzyme-Linked ImmunoSorbent Assay (ELISA)

*Purification of TSST-1: S. aureus* MN 8 was grown using the shake flask method, as described above. Supernatant from the sample was then subjected to alcohol precipitation, flatbed isoelectric focusing, and gel chromatography. It was determined that the purified TSST-1 preparation had a molecular weight average of 23kD as determined by sodium dodecyl sulfate-polyacrylamide gel electrophoresis, and an isoelectric point of 7.2 as determined by analytical isoelectric focusing. Purity was ascertained by polyacrylamide gel electrophoresis and serological analyses.

*Antibody to TSS-1:* Antisera were obtained by immunization of New Zealand White rabbits. TSST-1 was administered subcutaneously with complete Freund adjuvant on a weekly basis, starting at 10 µg and increasing to 50 µg by increments of 10 µg per week. Rabbits were tested for antibody 2 weeks after the last immunization and were bled if a high antibody titer to TSST-1 was demonstrable by double immunodiffusion. IgG was further prepared for use in the ELISA by precipitation of the serum three times with 35% ammonium sulfate and stored in PBS at-20°C.

*ELISA reagents:* Carbonate-bicarbonate buffer, pH 9.6, was used as the coating buffer. The diluent and washing solution was PBS, pH 7.4, containing 0.05% Tween 20 and 0.1% bovine serum albumin (PBS-Tween). The substrate solution was *p*-nitrophenyl phosphate (no. 104: Sigma Chemical Co., St. Louis, Mo.) (1 mg/mL) in 10% diethanolamine buffer, pH 9.8. All solutions contained 0.02% sodium azide.

*TSST*-*1*-*enzyme conjugation:* TSST-1 was conjugated to alkaline phosphatase (type VII-S: Sigma). A 100-ul volume of TSST-1 (10 mg/mL in PBS) was mixed with a 600-µl sample of alkaline phosphatase suspended in 3.2 M (NH₄)₂SO₄. After dialysis against PBS, 20 µl of 4% glutaraldehyde was added: the solution was mixed for 2 h, brought up to 2 mL with PBS, and again dialyzed against PBS. Insoluble material was eliminated by centrifugation.

TSST-1 conjugated to alkaline phosphatase was separated from unconjugated toxin and enzyme by gel chromatography over a column (2.6 by 40 cm) of Ultragel AcA 44 (LKB Instruments. Inc., Gaithersburg, Md.). Fractions (1 mL) were collected and analyzed for alkaline phosphatase activity and immunoreactivity. Alkaline phosphatase was detected by mixing 10 µl of 1:10 dilution of each fraction with 3.0 mL of substrate solution. Tubes were read by spectrophotometry at a wavelength of 405 nm after 5 to 10 min. at the same rate at which diluted fractions had been added. Immunoreactivity was determined with a microtiter plate previously coated with antibody to TSST-1 (see below). A 50-µl portion of each column fraction, diluted 1:4 in PBS-Tween, was added to a well and the plate was incubated overnight. Wells were washed, substrate solution was added, and color development was read at 405 nm on a microplate reader (model EL 307. Bio-tek Instruments. Burlington, Vt.). Fractions that contained alkaline phosphatase and were immunoreactive were pooled: bovine serum albumin and sodium azide were added at concentrations of 1.0 and 0.1%, respectively, and the conjugate was stored at 4°C. Conjugate thus prepared showed no evidence of decreased activity at 1 month.

*Immunoassay:* Nonperimeter wells of flat-bottomed, polystyrene microtiter plates (Immulon-2: Dynatech Laboratories, Inc. Alexandria, VA.) were coated with diluted TSST-1 antibody by incubating 200 µl of solution in all wells for 4 h at 3°C. Plates were then washed three times with PBS-Tween. Plates coated in batches were washed again with distilled water, dried over calcium sulfate, covered with plate sealers (Dynatech), and stored at 4°C until use. Plates prepared in this manner were stable for at least 1 month. The optimal dilution of coating antibody was determined by checkerboard titration with TSST-1 conjugate. Final concentrations of both reagents were chosen to yield an optimal density of 1.0 at 60 min after the addition of substrate solution.

Culture supernatants to be assayed were diluted in PBS-Tween. At least three dilutions of each supernatant were tested to assure that the TSST-1 concentration would fall within the range of the standard curve. Samples (100 µl) of each dilution were added to coated wells in duplicate, followed by 100 µl of appropriately diluted TSST-1 conjugate. A standard curve was run in duplicate on each plate with unlabeled TSST-1 at concentrations indicated below. After overnight incubation at room temperature in a humidified box, wells were again rinsed three times with PBS-Tween: 200 µl of prewarmed substrate solution was added, and the plate was incubated at 37°C. Color development was quantitated by spectrophotometry at 405 nm on the microplate reader when wells containing uninhibited TSST-1 conjugate reached an optical density of 1.0, usually after about 60 min.

A standard curve for each experiment was established by plotting optical density as a function of the logarithm of TSST-1 concentration. The correlation coefficient of the linear portion of the curve was derived on a programmable calculator. For sample dilutions with optical densities within the linear portion of the curve, the log of TSST-1 concentration was derived from the mean of duplicate optical density readings. Taking the antilog and correcting for same dilution provided the TSST-1 concentration in the culture supernatant.

TSST-1 concentrations are expressed in micrograms per milliliter to the nearest decimal place. Reproducibility is expressed in terms of the 95% confidence intervals of the mean, based upon the standard deviation (SD) of the log of TSST-1 concentrations (95% confidence interval = mean = 1.96 (SD/√n)].

The above described ELISA methodology is described in the journal article, Competitive, Enzyme-Linked ImmunoSorbent Assay for Toxic Shock Syndrome Toxin 1; Journal of Clinical Microbiology, July 1985, Vol. 22 No. 1, p. 26-31.

**TABLE 1**

| Compound | Ca (mM) added to shake flask | % TSST-1 Change (µg/mL) vs. control | % TSST-1 Change (µg/ml/mM) vs. control |
|---|---|---|---|
| Comparative Examples | | | |
| calcium chloride | 15.1 | 37 | 2.5 |
| calcium stearate* | 9.9 | 65 | 6.6 |
| calcium lactate | 8.8 | 49 | 5.6 |
| calcium citrate malate | 6.8 | 89 | 13.1 |

| Calcium Sugar Acid Salts | | | |
|---|---|---|---|
| calcium glycerate | 4.0 | 68 | 17.0 |
| calcium gluconate | 3.8 | 81 | 21.3 |
| calcium α-D-heptagluconate | 3.8 | 47 | 12.5 |

| | | | |
|---|---|---|---|
| * calcium stearate solid is added directly into the media | | | |

This example demonstrates that the calcium sugar acid salts -calcium glycerate, calcium gluconate, calcium α-D-heptagluconate- reduced TSST-1 production (calcium glycerate-68%, calcium gluconate-81%, calcium α-D-heptagluconate-47%), as compared to the control of PBS and BHI, by *S. aureus* at low concentrations.

### EXAMPLE 2

This example shows the ability of calcium sugar acid salts to almost completely disrupt TSST-1 production at higher concentrations. In addition, the example demonstrates that at higher concentrations calcium sugar acid salts are more effective at reducing TSST-1 production than other calcium salts.

### Materials and Methods

The materials and methods used (*Shake Flask Method and ELISA)* were the same as those used for EXAMPLE 1.

**TABLE 2**

| Compound | Ca (mM) added to shake flask | % TSST-1 Change (µg/ml) vs. control | % TSST-1 Change (µg/ml/mM) vs. control | Solubility (mM of Calcium in H₂O at 25°C) |
|---|---|---|---|---|
| Comparative Examples (Calcium Salts) | | | | |
| calcium chloride | 75.6 | 82 | 1.1 | 7324 |
| calcium stearate* | 98.8 | 62 | 0.6 | <0.003 |
| calcium lactate | 53.1 | 89 | 1.7 | 229 |
| calcium citrate malate | 22.6 | 88 | 3.9 | 45.1 |

| Calcium Sugar Acid Salts | | | | |
|---|---|---|---|---|
| calcium glycerate | 39.7 | 99 | 2.5 | 349 |
| calcium gluconate | 38.4 | 98 | 2.5 | 78 |
| calcium α-D-heptagluconate | 38.1 | 98 | 2.6 | 190 |

| | | | | |
|---|---|---|---|---|
| * calcium stearate solid is added directly into the media | | | | |

This example demonstrates (1) that calcium sugar acid salts -calcium glycerate, calcium gluconate, calcium α-D-heptagluconate- at higher concentrations of about 40 mM Ca (as compared to the concentrations of Table 1 of about 4.0 mM Ca) almost completely disrupt TSST-1 production with about a 98% reduction of TSST-1 production, as compared to the control of PBS and BHI; (2) that calcium sugar acid salts -calcium glycerate, calcium gluconate, calcium α-D-heptagluconate- at concentrations of about 40 mM Ca reduce TSST-1 production to a greater degree (about 98%) as compared to other calcium salts (calcium chloride-82%, calcium stearate-62%, calcium lactate-89%, calcium citrate malate-88%); (3) that the calcium sugar acid salts have a greater solubility (calcium glycerate 349 mM, calcium gluconate 81.3 mM, calcium α-D-heptagluconate 190 mM) than calcium citrate malate (45.1 mM) and calcium stearate (<0.003 mM), it is believed the higher solubility of the calcium sugar acid salts is one possible reason for the greater degree of TSST-1 reduction (about 98%) as compared to the TSST-1 reduction of calcium citrate malate (88%) and calcium stearate (62%) whose TSST-1 reduction levels remained about the same as compared to their TSST-1 reduction at lower concentrations as shown in Table 1 (calcium citrate malate at 6.8 Mm Ca reduced TSST-1 production by 89%, and at 22.6 Mm Ca reduced TSST-1 production by 88% and calcium stearate at 9.9 Mm Ca reduced TSST-1 production by 65%, and at 98.8 Mm Ca reduced TSST-1 production by 62%).

While not being limited to theory, it is believed that in addition to greater solubility (as compared to calcium stearate and calcium citrate malate) the chemical structure of the calcium sugar acid salts provides a greater reduction in TSST-1 than (a) calcium chloride which lacks a sugar acid salt anion; (b) calcium stearate as calcium stearate comprises a carbon chain of a much greater length (C₁₈) than most of the calcium sugar acid salts (C₃-C₁₄), reducing solubility; (c) calcium lactate and calcium citrate malate, the additional (CHOH) in the sugar acid salt is believed to be responsible for the greater degree of TSST-1 reduction (about 98% as compared to TSST-1 reduction of calcium lactate (89%) or calcium chloride (82%).

### EXAMPLE 3

This example demonstrates that calcium sugar acid salts are more effective at reducing TSST-1 production by S. *aureus* than sodium sugar acid salts.

### Materials and Methods

The materials and methods used (*Shake Flask Method and ELISA)* were the same as those used for EXAMPLE 1.

**TABLE 3**

| Compound | Anion (mM) added to shake flask | % TSST-1 Change (µg/ml) vs. control |
|---|---|---|
| sodium gluconate | 40.0 | 27% reduction |
| calcium gluconate | 7.6 | 81% reduction |

This example demonstrates that calcium sugar acid salts, in this instance calcium gluconate, are more effective at reducing TSST-1 production than sodium sugar acid salts, in this instance sodium gluconate, as demonstrated in Table 3, wherein calcium gluconate at a concentration of 7.6 mM reduced TSST-1 production by 81%, as compared to sodium gluconate at a concentration of 40.0 mM which reduced TSST-1 production by only 27%.

### EXAMPLE 4

This example shows that the D-form of calcium sugar acid salts is more effective at reducing TSST-1 production by S. *aureus* than the L-form of calcium sugar acid salts.

### Materials and Methods

The materials and methods used (*Shake Flask Method and ELISA)* were the same as those used for EXAMPLE 1.

**TABLE 4**

| Compound | Ca (mM) added to shake flask | % TSST-1 Change (µg/ml) vs. control | % TSST-1 Change (µg/ml/mM) vs. control |
|---|---|---|---|
| calcium D-glycerate | 4.0 | 92 | 22.9 |
| calcium L-glycerate | 4.0 | 61 | 15.3 |
| calcium DL-glycerate | 4.0 | 68 | 17.0 |

This example shows that the D-form of a calcium sugar acid salt, in this instance calcium D-glycerate, at an equal amount to the L-form of a calcium sugar acid salt (calcium L-glycerate) of about 4.0 mM, reduces TSST-1 production by *S* .*aureus* by a greater amount (about 92%) as compared to calcium L-glycerate (about 61%). Further, when calcium DL-glycerate is tested the reduction in TSST-1 production is increased (about 68%) over the calcium L-glycerate alone (about 61%); once again demonstrating the greater TSST-1 reduction of the D-form of a calcium sugar acid salt as compared to the L-form of a calcium sugar acid salt.

### EXAMPLE 5

This example shows that calcium sugar acid salts have the ability to almost completely disrupt TSST-1 production while being substantially non-lethal to S. *aureus*.

### Materials and Methods

The materials and methods used (*Shake Flask Method and ELISA)* were the same as those used for EXAMPLE 1.

**TABLE 5**

| Calcium Salt | Ca (mM/mL) Concentration added to shake flask | % TSST-1 Change (µg/ml) vs. control | Change in S. *aureus* cfu/mL vs. control |
|---|---|---|---|
| calcium glycerate | 39.7 | 99 | <2 log |
| calcium gluconate | 38.4 | 98 | <2 log |
| calcium α-D-heptagluconate | 38.1 | 98 | <2 log |
| calcium D-glycerate | 39.7 | 99 | <2 log |

This example demonstrates that calcium sugar acid salts, such as calcium glycerate, calcium gluconate, calcium α-D-heptagluconate, and calcium D- glycerate are able to reduce TSST-1 production (all reduced TSST-1 production by about 98% as compared to controls) while being substantially non-lethal to *S. aureus*.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A feminine article comprising calcium sugar acid salt, wherein the calcium sugar acid salt reduces *S. aureus* TSST-1 production by at least about 70%, preferably by at least 80%, 90%, or 95%, as measured by ELISA, as compared to TSST-1 production by *S. aureus* to which no calcium sugar acid salt has been added, wherein the calcium sugar acid salt has a solubility that provides a Ca concentration of at least about 50 millimoles/L of water at 25°C;
wherein the feminine article is an absorbent article comprising one or more absorbent materials that absorb or contain a bodily fluid; and
wherein the calcium sugar acid salt is added to fiber forming the absorbent material.

2. The feminine article of claim 1, wherein the calcium sugar acid salt comprises: 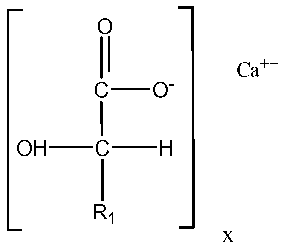 Wherein:
R₁= (CHOH)ₙCH₂OH; (CHOH)ₙCHO; (CH₂OH)ₙCOO⁻; (CHOH)ₙCH₂(O)R₂
R₂= H; (CHOH)ₘCH₂OH
n=0-4
m=0-3
m+n= 0-3
x= 1 or 2

3. The feminine article of claim 1 or 2, wherein the calcium sugar acid salt is at least one of a calcium glucarate, calcium gluconate, calcium heptagluconate, calcium diketogluconate, calcium glycerate, calcium fructonate, calcium altronate, calcium mannonate, calcium gulonate, calcium galactonate, calcium ribonate, calcium arabinonate, calcium xylonate, calcium erythronate, calcium sorbonate, calcium ribulonate, and calcium xylulonate.

4. The feminine article of any of the preceding claims, wherein the calcium sugar acid salt is substantially non-lethal to *S. aureus* when measured by the Shake Flask Method.

5. The feminine article of any of the preceding claims, wherein the reduction of TSST-1 production is determined by *S. aureus* grown using the shake flask method.

6. The feminine article of any of the preceding claims, wherein the feminine article is at least one of a tampon, sanitary napkin, intralabial device, incontinence article, or liner.

7. The feminine article of any of the preceding claims, wherein the calcium sugar acid salt is a D-form calcium sugar acid salt.

8. The feminine article of claim 7, wherein the D-form calcium sugar acid salt is at least one of a calcium glucarate, calcium gluconate, calcium heptagluconate, calcium diketogluconate, calcium glycerate, calcium fructonate, calcium altronate, calcium mannonate, calcium gulonate, calcium galactonate, calcium ribonate, calcium arabinonate, calcium xylonate, calcium erythronate, calcium sorbonate, calcium ribulonate, and calcium xylulonate.

## Patentansprüche

1. Damenartikel, umfassend Calciumzuckersäuresalz, wobei das Calciumzuckersäuresalz die *S*.-*aureus*-TSST-1-Produktion um mindestens etwa 70 % reduziert, vorzugsweise um mindestens 80 %, 90 % oder 95 %, gemessen durch ELISA, verglichen mit der TSST-1-Produktion durch *S. aureus*, zu welchem kein Calciumzuckersäuresalz hinzugefügt worden ist, wobei das Calciumzuckersäuresalz eine Löslichkeit in Wasser aufweist, die eine Calciumkonzentration von mindestens 50 Millimol/l bei 25 °C ermöglicht,
wobei der Damenartikel ein Absorptionsartikel ist, umfassend ein oder mehrere Absorptionsmaterialien, welche eine Körperflüssigkeit absorbieren oder enthalten können, und
wobei das Calciumzuckersäuresalz zu Fasern hinzugefügt wird, um das Absorptionsmaterial zu bilden.

2. Damenartikel nach Anspruch 1, worin das Calciumzuckersäuresalz umfasst: 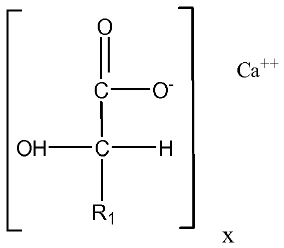 worin:
R₁= (CHOH)ₙCH₂OH, (CHOH)ₙCHO, (CH₂OH)ₙCOO-, (CHOH)ₙCH₂(O)R₂
R₂= H, (CHOH)ₘCH₂OH
n=0-4
m=0-3
m+n= 0-3
x= 1 oder 2

3. Damenartikel nach Anspruch 1 oder 2, worin das Calciumzuckersäuresalz mindestens eines von einem Calciumglucarat, Calciumgluconat, Calciumheptagluconat, Calciumdiketogluconat, Calciumglycerat, Calciumfructuronat, Calciumaltronat, Calciummannonat, Calciumgulonat, Calciumgalactonat, Calciumribonat, Calciumarabinonat, Calciumxylonat, Calciumerythronat, Calciumsorbonat, Calciumribulonat und Calciumxylulonat ist.

4. Damenartikel nach einem der vorstehenden Ansprüche, worin das Calciumzuckersäuresalz im Wesentlichen nicht lethal für *S. aureus* ist, wenn nach dem Schüttelkolbenverfahren gemessen wird.

5. Damenartikel nach einem der vorstehenden Ansprüche, worin die Reduzierung der TSST-1-Produktion anhand von *S. aureus* bestimmt wird, gezüchtet mittels Anwendung des Schüttelkolbenverfahrens.

6. Damenartikel nach einem der vorstehenden Ansprüche, wobei der Damenartikel mindestens eines von einem Tampon, Damenbinde, intralabiale Vorrichtung, Inkontinenzartikel oder Einlage ist.

7. Damenartikel nach einem der vorstehenden Ansprüche, worin das Calciumzuckersäuresalz ein Calciumzuckersäuresalz der D-Form ist.

8. Damenartikel nach Anspruch 7, worin das Calciumzuckersäuresalz der D-Form mindestens eines von einem Calciumglucarat, Calciumgluconat, Calciumheptagluconat, Calciumdiketogluconat, Calciumglycerat, Calciumfructuronat, Calciumaltronat, Calciummannonat, Calciumgulonat, Calciumgalactonat, Calciumribonat, Calciumarabinonat, Calciumxylonat, Calciumerythronat, Calciumsorbonat, Calciumribulonat und Calciumxylulonat ist.

## Revendications

1. Article féminin comprenant un sel calcique d'acide de sucre, dans lequel le sel calcique d'acide de sucre réduit la production de TSST-1 de *S. aureus* d'au moins environ 70 %, de préférence, d'au moins 80 %, 90 % ou 95 %, comme mesuré par ELISA, par comparaison avec la production de TSST-1 par un *S. aureus* auquel aucun sel calcique d'acide de sucre n'a été ajouté, dans lequel le sel calcique d'acide de sucre a une solubilité qui fournit une concentration en Ca d'au moins environ 50 millimoles/L d'eau à 25 °C ;
où l'article féminin est un article absorbant comprenant un ou plusieurs matériaux absorbants qui absorbent ou contiennent un fluide corporel ; et
dans lequel le sel calcique d'acide de sucre est ajouté à la fibre formant le matériau absorbant.

2. Article féminin selon la revendication 1, dans lequel le sel calcique d'acide de sucre comprend : 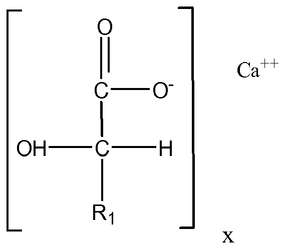 où :
R¹= (CHOH)ₙCH₂OH ; (CHOH)ₙCHO ; (CH₂OH)ₙCOO⁻ ; (CHOH)ₙCH₂(O)R₂
R₂= H ; (CHOH)ₘCH₂OH
n=0-4
m=0-3
m+n= 0-3
x= 1 ou 2

3. Article féminin selon la revendication 1 ou 2, dans lequel le sel calcique d'acide de sucre est au moins un sel parmi le glucarate de calcium, le gluconate de calcium, l'heptagluconate de calcium, le dicétogluconate de calcium, le glycérate de calcium, le fructonate de calcium, l'altronate de calcium, le mannonate de calcium, le gulonate de calcium, le galactonate de calcium, le ribonate de calcium, l'arabinonate de calcium, le xylonate de calcium, l'érythronate de calcium, le sorbonate de calcium, le ribulonate calcium et le xylulonate de calcium.

4. Article féminin selon l'une quelconque des revendications précédentes, dans lequel le sel calcique d'acide de sucre est essentiellement non létal pour le *S. aureus* lorsqu'on mesure par le procédé de flacon sous agitation.

5. Article féminin selon l'une quelconque des revendications précédentes, dans lequel la réduction de production de TSST-1 est déterminée par *S. aureus* mis en croissance en utilisant le procédé de flacon sous agitation.

6. Article féminin selon l'une quelconque des revendications précédentes, où l'article féminin est au moins un article parmi un tampon, une serviette hygiénique, un dispositif intralabial, un article pour l'incontinence ou un protège-slip.

7. Article féminin selon l'une quelconque des revendications précédentes, dans lequel le sel calcique d'acide de sucre est un sel calcique d'acide de sucre de forme D.

8. Article féminin selon la revendication 7, dans lequel le sel calcique d'acide de sucre de forme D est au moins un parmi le glucarate de calcium, le gluconate de calcium, l'heptagluconate de calcium, le dicétogluconate de calcium, le glycérate de calcium, le fructonate de calcium, l'altronate de calcium, le mannonate de calcium, le gulonate de calcium, le galactonate de calcium, le ribonate de calcium, l'arabinonate de calcium, le xylonate de calcium, l'érythronate de calcium, le sorbonate de calcium, le ribulonate calcium et le xylulonate de calcium.
